Europäisches Patentamt

**European Patent Office** (11) Publication number: **0 122 664**

Office européen des brevets **A2**

(12) **EUROPEAN PATENT APPLICATION**

(21) Application number: **84200477.2**

(22) Date of filing: **05.04.84**

(51) Int. Cl.³: **C 11 D 1/14**
C 11 D 3/04, A 61 L 9/01
E 03 D 9/03

(30) Priority: **14.04.83 US 484778**

(43) Date of publication of application:
**24.10.84 Bulletin 84/43**

(84) Designated Contracting States:
**AT BE CH DE FR GB IT LI NL SE**

(71) Applicant: **THE PROCTER & GAMBLE COMPANY**
**301 East Sixth Street**
**Cincinnati Ohio 45202(US)**

(72) Inventor: **Wong, Louis F.**
**9868 Bobwhite Pl.**
**Mason, Ohio 45040(US)**

(72) Inventor: **Sterling, Rosstain Freestan**
**5787 Tall Oak Drive**
**Milford, Ohio 45150(US)**

(72) Inventor: **Borcher, Thomas Andrew, Sr.**
**Box 137D Burger Road**
**Melbourne Kentucky 41059(US)**

(74) Representative: **Ernst, Hubert et al,**
**PROCTER & GAMBLE EUROPEAN TECHNICAL CENTER**
**Temselaan 100**
**B-1820 Strombeek-Bever(BE)**

(54) Alkali earth metal salt/alkali metal surfactant dry mix cakes for longer lasting dosing dispenser.

(57) Solid cakes comprising a water-soluble alkali earth metal salt and a selected alkali metal surfactant. The stoichiometric ration of the salt to surfactant ranges from about 0.1:1 to 2:1. Perfume, dye or salt, or any combination thereof can be added. The cakes have improved chemical stability and improved longevity. Preferred cakes are made with about 75% of a dry mix of magnesium sulfate and sodium alkyl sulfate having a stoichiometric ratio of 0.5:1, 18% perfume, 3% free fatty alcohol, and 2.7% dye.

# ALKALI EARTH METAL SALT/ALKALI METAL SURFACTANT DRY MIX CAKES FOR LONGER LASTING DOSING DISPENSER

Louis F. Wong

Rosstain F. Sterling

Thomas A. Borcher, Sr.

## TECHNICAL FIELD

The present invention relates in general to a solid surfactant cake. The cakes are particularly useful in conjunction with a toilet tank dosing dispenser which automatically dispenses a ration of surfactant, and optionally perfume, and/or dye, etc. to the bowl of a toilet, responsive to the flushing of the toilet.

## BACKGROUND

In treating toilet flush water with chemicals in order to produce desirable effects such as bowl aesthetics, cleaning, disinfection, deodorization, aerosol reduction, etc., it is desirable that the chemicals be dispensed into the flush water automatically each time the toilet is flushed. The prior art discloses numerous devices which have been designed for this purpose. Exemplary of such devices are disclosed in:

U.S. Pat. No. 4,171,546, Dirksing, issued Oct. 23, 1979;

U.S. Pat. No. 4,186,856, Dirksing, issued Feb. 5, 1980;

U.S. Pat. No. 4,200,606, Kitko, issued April 29, 1980;

U.S. Pat. No. 4,208,747, Dirksing, issued June 24, 1980;

U.S. Pat. No. 4,216,027, Wages, issued August 5, 1980;

U.S. Pat. No. 4,246,129, Kacher, issued Jan. 20, 1981;

U.S. Pat. No. 4,247,070, Dirksing, issued Jan. 27, 1981;

U.S. Pat. No. 4,248,827, Kitko, issued Feb. 3, 1981;

U.S. Pat. No. 4,251,012, Williams et al., issued Feb. 17, 1981;

U.S. Pat. No. 4,253,951, McCune, issued March 3, 1981;

U.S. Pat. No. 4,281,421, Nyquist et al., issued Aug. 4, 1981;

U.S. Pat. No. 4,283,300, Kurtz, issued Aug. 11, 1981;

U.S. Pat. No. 4,302,350, Callicott, issued Nov. 24, 1981;

British Patent Appl. No. 2.116.040, Mueller et al., published September 21, 1983; and

European Pat. Appln. 0,005,286, Nyquist, published Nov. 14, 1979, all of which are incorporated herein by reference.

Particularly desirable devices are those used in conjunction with a solid cake composition. In this type of device a measured amount of water enters the device during one flush cycle and remains in contact with the cake between flushes, thereby forming a concentrated solution of the composition which is dispensed into the flush water during the next flush. The advantages of such devices are that the chemical composition can be packaged and shipped in more concentrated form than aqueous solutions of the chemicals. Also, the problems of liquid spillage resulting from breakage of the dispensers during shipment or handling is eliminated. Especially preferred devices for automatic dispensing of chemicals from solid cake compositions into the toilet are those described in U.S. Pat. No. 4,171,546, Dirksing, issued October 23, 1979; U.S. Pat. No. 4,208,747, Dirksing, issued June 24, 1980; U.S. Pat. No. 4,186,856, Dirksing, issued February 5, 1980; all of which are incorporated by reference. A preferred version of the dispenser is used in BRIGADE[R], an automatic toilet bowl cleaner sold by The Procter & Gamble Company.

Prior art surfactant cake compositions used in the "Dirksing" dispensing devices are disclosed in U.S. Pat. No. 4,308,625, Kitko, issued January 5, 1982; U.S. Pat. No. 4,310,434, Choy and Greene, issued January 12, 1982; and U.S. Pat. No. 4,278,5671, Choy, issued July 14, 1981, entitled "Surfactant Cake Compositions;" all of which are incorporated herein by reference. The surfactants provide cleaning and sudsing in the toilet bowl and also serve to dispense other components of the compositions such as dyes, perfumes, organic resins, etc. Anionic surfactants, especially the organic sulfates and sulfonate types, are used in these compositions because of their availability, low cost and excellent cleaning and dispensing properties.

Water-soluble inert salts such as alkali metal chlorides and sulfates are used in such compositions to act as a "processing aid" so that the composition can be formed into cakes of desirable size without using excessive amounts of active ingredients. The predominant ingredients of the cake compositions are usually the surfactant, perfume, dye, and the processing aid salt. Anionic, nonionic, ampholytic, zwitterionic or cationic surfactants are used. The surfactant or surfactant mixture should be solid at temperatures up to about 100°F (40°C). Anionics and nonionics and mixtures thereof are useful. Anionics are the most preferred.

The prior art anionic surfactant cakes can be described as essentially the water-soluble alkali metal salts, of organic sulfuric reaction products having in their molecular structure an alkyl or an alkylaryl radical containing from 8 to 22 carbon atoms.

A major problem in this art has been short and/or erratic longevity of surfactant cakes during use, caused by too high a solubility rate or by uneven solubility. Another problem with cakes using solid alkali earth metal alkyl sulfate surfactants is stabilizing them against chemical degradation during processing, storage and use.

Another problem is related to the incorporation of higher levels of perfume into surfactant cake formulations while maintaining desired firmness.

## SUMMARY OF THE INVENTION

The present invention is directed to stable solid water-soluble cake compositions which comprise a dry mix of a water-soluble alkali earth metal salt and a selected anionic alkali metal surfactant. Preferably the cake has from 20% up to 100% of the dry mix; from 0% to 30% perfume; from 0% to 40% inert salt, 0% to 15% free fatty alcohol, and from 0% to 12% dye. When formulated for use in a dosing dispenser for flush toilet, these cakes preferably have at least about 10% of any combination of perfume and/or dye. The preferred dry mix is made, e.g., with magnesium sulfate and sodium lauryl sulfate at a stoichiometric ratio of 0.1:1 to 2:1.

A major object of the present invention is to provide a long lasting surfactant cake, having excellent chemical stability, which

0122664

- 4 -

does not require preparation of a predried alkali earth metal surfactant prior to manufacture of the cake.

Another object of the present invention is to provide a surfactant cake for an automatic dosing dispenser which has improved stability and longevity. Another object of the present invention is to provide a surfactant cake formulation which can include a higher level of perfume while maintaining firmness and improved longevity.

Yet another object of the present invention is to provide an improved method of making a more stable surfactant cake for a dosing dispenser.

## DETAILED DESCRIPTION OF THE INVENTION

According to the present invention it has been found that superior cakes can be made with certain dry mixed anionic organic surfactants and water-soluble alkali earth metal salts. The detergent cakes are especially useful in dosing dispensers for toilets. These new cakes have controlled solubility and chemical stability so as to perform their required functions and have improved longevity over surfactant cakes in which an alkali metal surfactant salt is formulated only with alkali metal salts.

The water-soluble alkali metal salts of anionic surfactants are preferably very soluble. In formulating anionic surfactant-containing cakes, water-soluble alkali earth metal salt is dry mixed with selected alkali metal anionic surfactant at a salt to surfactant stoichiometric ratio of 0.1:1 to 2:1, preferably 0.3:1 to 1:1.

The term "dry mix" as used herein means the mixture of alkali earth metal salt and anionic surfactant of the invention in the stoichiometric ratio of salt:surfactant of 0.1:1 to 2:1.

The following description of the invention, and the manner and process of making and using it, is set forth using magnesium salt and $C_{12}$ to $C_{14}$ sodium alkyl sulfate surfactant as the preferred embodiment. It will be understood, however, that other embodiments using different alkali earth metal salts and other anionic surfactants of the present invention can be used.

Cakes preferably contain the dry mix at levels of 20% up to 100%, more or less, of the total cake content. The dry mix is more preferably present at 50% to 90%.

All percentages and ratios herein are "by weight" unless specified otherwise. For the purposes of the present invention, "water-soluble" means having a solubility in water of at least 1 gram per 100 grams of water at 20°C.

The compositions herein will be described with particular reference to their use in conjunction with dispensers which dispense chemicals into the flush water of toilets, although it is to be understood that said compositions can be used in other applications where a solid cake surfactant composition is desired.

The new cake compositions preferably comprise from about 50% to about 90% of a dry mix made of a water-soluble alkali earth metal salt and alkyl sulfate surfactant wherein the alkyl group has a carbon chain length of from 8 to 22 carbon atoms, preferably from 10 to 16 carbon atoms. Preferred are water-soluble salts from the group of magnesium, calcium, and barium metal chlorides, sulfates and nitrates. The cakes of this invention preferably contain at least 1.5% of dye and/or perfume. The more preferred cake compositions contain from about 75% of the dry mix, from 10% to 20% perfume, from 0.5% to 15% of a fatty alcohol, and from 1.5% to 5% dye. The cakes preferably weigh from 20 grams to 80 grams and have a density of 0.8 to 1.8 gm/cc.

Cakes of the invention desirably have a pH of from 6 to about 10. Preferably the pH is from 7 to 9.5.

The cakes of this invention preferably have moisture contents of 0.1% to 10%, more preferably 1% to 5%.

### Alkali Earth Metal Salt

An essential component of the cake composition is a water-soluble alkali earth metal salt, i.e., a metal of Group IIA of the Periodic Chart of Elements. The salt can be inorganic or organic. Preferred are the sulfates, chlorides and nitrates of magnesium, calcium and barium. Anhydrous and hydrous salts

- 6 -

can be used. Examples of specific salts are: calcium acetate, magnesium acetate, magnesium sulfate, magnesium chloride, magnesium nitrate, calcium sulfate, calcium chloride, calcium nitrate, barium chloride, barium nitrate, magnesium benzoate, magnesium salicylate, magnesium thiosulfate, calcium chlorate, barium acetate, barium aluminate, barium bromide, calcium bromide, calcium bisulfite and calcium acrylate.

Without being bound by theory, we believe that the alkali earth metal salt does not react with the alkali metal anionic surfactant in the dry cake state. When the cake is exposed to water, the alkali earth metal anionic surfactant is formed. This species of surfactant is less soluble than the alkali metal form of the surfactant.

Because of this reduced solubility the longevity of the cake is increased over a cake made with alkali metal surfactant and only alkali metal salt.

### The Surfactant

The other essential component of the cake composition is a selected anionic surfactant. The surfactant or surfactant mixture should be solid at ambient temperature, i.e., temperatures up to about 40°C.

The anionic surfactants of the invention can be broadly described as the water-soluble alkali metal salts, of organic sulfuric acid reaction products having in their molecular structure an alkyl chain containing from about 8 to about 22 carbon atoms and a radical selected from the group consisting of sulfonic acid and sulfuric acid ester radicals. Important examples of the anionic surfactants which can be employed in the practice of the present invention are the sodium, lithium, or potassium alkyl sulfates, especially those obtained from higher alcohols ($C_8$ to $C_{22}$ carbon atoms), sodium, lithium, or potassium alkyl benzene sulfonates, in which the alkyl group contains from about 9 to about 15 carbon atoms, (the alkyl radical can be a straight or branched aliphatic chain); paraffin sulfonate surfactants having the general formula $RSO_3M$, wherein R is a primary, secondary or tertiary alkyl group containing from about 8 to about 22 carbon atoms (preferably 10 to 18 carbon atoms) and M is an alkali metal, e.g., sodium, lithium, or potassium; sodium alkyl glyceryl ether sulfonates, especially those ethers of the higher alcohols derived from tallow and coconut oil; sodium coconut oil fatty acid monoglyceride sulfates and sulfonates; sodium, lithium, or potassium salts of sulfuric acid esters of the reaction product of one mole of a higher fatty alcohol (e.g., tallow or coconut oil alcohols) and about 1 to 10 moles of ethylene oxide; sodium, lithium, or potassium salts of alkyl phenol ethylene oxide ether sulfates with about 1 to about 10 units of ethylene oxide per molecule and in which the alkyl radicals contain from about 8 to about 12 carbon atoms; the reaction products of fatty acids esterified with isethionic acid and neutralized with alkali metal hydroxide where, for example, the fatty acids are derived from coconut oil; sodium, lithium or potassium salts of fatty acid amides of a methyl tauride in which the fatty acids, for example, are derived from coconut oil, and sodium, lithium or potassium β-acetoxy- or β-acetamido- alkanesulfonates where the alkane has from 8 to 22 carbon atoms.

## Suggested Steps for Making and Using the Preferred Cakes of This Invention

1.  Charge an amalgamator with an alkali metal alkyl sulfate surfactant (e.g., sodium lauryl sulfate).

2.  Add with mixing an alkali earth metal salt (e.g., magnesium sulfate) in an amount which is in a stoichiometric ratio of about 0.1:1 to 2:1 of salt to surfactant.

3.  Mix until uniform.

4.  Add optional ingredients such as perfume, dyes, free fatty alcohol etc. Amalgamate.

5.  Plod the mixture of Step 4 under vacuum and then extrude into a log of about 4.9 cms x 1.3 cms using a Mazzoni Model Duplex B-100 Plodder, Busto Arsizio, Italy, having a nozzle size of 4.9 cms x 1.35 cms. Plodder barrel temperature 20-27°C; nose (nozzle) temperature not controlled.

6.  Cut the log of Step 5 into cakes of 8.6 cms.

7.  Place the cakes into dosing dispensers and seal.

Of course, cakes can also be made by dry mixing and compressing using conventional tabletting equipment, such as a Carver Laboratory Press, Model C, Fred S. Carver, Inc., subsidiary of Sterling, Inc., Menomoner Falls, Wisconsin 53051.

Perfumes

Perfume is a highly desirably ingredient for surfactant cake compositions. Cakes are made with perfume at levels of from 0% to 30%, but levels of 5% to 25% are preferred, and 10% to 20% levels are most preferred. In U.S. Pat. No. 4,246,129, Kacher, issued January 20, 1981 (incorporated herein by reference), certain perfume materials are disclosed which perform the added function of reducing the solubility of anionic sulfonate and sulfate surfactants. At higher levels of perfumes, e.g., over 12% in anionic surfactant cakes which are not formulated in accordance with the present invention, softness (i.e., lack of firmness) of the cake can be a problem.

Firmness of the cake is measured at a temperature of about 25°C by the use of a penetrometer. Acceptable firmness is represented by penetrometer readings of from 45 to 150 and preferably from 90 and 120 using a Lab-Line Universal Hi-Accuracy Penetrometer (0.1 mm divisions) equipped with wax penetration needle ASTM D1321 (Lab-Line Instruments, Inc., Melrose Park, Illinois), Cat. No. 4101.

The instrument is operated in the following manner. Level the base and place a 150 g. weight on the plunger top. Cut a cake in half with a knife. Place the cake with a fresh cut end beneath the penetrometer needle, raised to the zero position. Lower needle (via elevator screw) until needle just touches cut end of the cake. Depress the trigger for 10 seconds (the needle will lower into the cake), then release. To read firmness, lower the depth gauge bar until it just touches plunger.

Firmness readings are taken directly from the gauge, in units of tenths of millimeters.

Raise the needle to zero position, remove the cake, and record cake firmness.

## Optional Inert Salts

The inert salts can be used as fillers and/or as processing aids in the compositions of the present invention. They can be any water-soluble inorganic or organic salt or mixtures of such inert salts which do not destabilize the cake. Preferably they are alkali metal salts. Examples of suitable inert salts include various alkali metal sulfates, chlorides, borates, bromides, citrates, acetates, lactates, etc.

Specific examples of suitable salts include sodium sulfate, sodium chloride, potassium sulfate, lithium chloride, lithium sulfate, sodium borate, potassium bromide, sodium citrate, and sodium acetate. The preferred inert salts are inorganic salts preferably the alkali metal sulfates and chlorides. Particularly preferred salts, because of their low cost are sodium sulfate and sodium chloride. The inert salts are present in the compositions up to 70% and higher, but preferably are present at a level of 0% to 20%.

Inert salts are used as fillers and/or processing aids, particularly to formulate cakes with higher levels of perfume and/or lower levels of the dry mix of anionic surfactant and alkali earth metal salt.

## The Dyes

Dyes may be included at levels of from about 0.5% to 12%, preferably 1.5% to 5%. Dyes and perfumes can be used together in the cakes of the invention or either one can be used alone. Examples of suitable dyes are Alizarine Light Blue B (C.I. 63010), Carta Blue VP (C.I. 24401), Acid Green 2G (C.I. 42085), Astrogen Green D (C.I. 42040), Supranol Cyanine 7B (C.I. 42675), Maxilon Blue 3RL (C.I. Basic Blue 80), Drimarine Blue Z-RL (C.I. Reactive Blue 18), Alizarine Light Blue H-RL (C.I. Acid Blue 182), FD&C Blue No. 1 and FD&C Green No. 3. (See the patents of Kitko, U.S. Pat. No. 4,200,606, issued April 29, 1980, and U.S. Pat. No. 4,248,827, issued February 3, 1981, both incorporated herein by reference.) C.I. refers to Color Index.

### Free Fatty Alcohols

Fatty alcohols reduce the solubility of the cakes herein, and therefore provide an additional increment of improvement in longevity. Generally from 0.5% to 15% coconut fatty alcohol is used; 1% to 10% is preferred and 2% to 6% is most preferred.

Fatty alcohols having carbon chain lengths of 8 to 18 are preferred; more preferred are those with 10 to 16 carbon chain lengths.

### Other Optional Ingredients

Various other optional materials may be included in the compositions herein. See U.S. Pat. No. 4,278,571, Choy, issued July 14, 1981, for an extensive list of them.

### Use in Combination with A Bleach Cake

The present surfactant cake can be used in combination with a separate bleach cake. The preferred bleach cake comprises 95% HTH and 5% lithium sulfate.

The cake is formed into shapes with dimensions appropriate to fit the cake compartment of the gravity feed dosing dispenser which holds the cake.

### Dispensing Means

Dispensing means which can be used to dispense compositions of the present invention into the toilet flush water are exemplified by those described in U.S. Pat. Nos. 3,831,205, 3,341,074, 3,504,384, 2,688,754, 4,036,407, 4,171,546, 4,208,747, and 4,186,856, above noted. Details of the preferred dispensing means is described below.

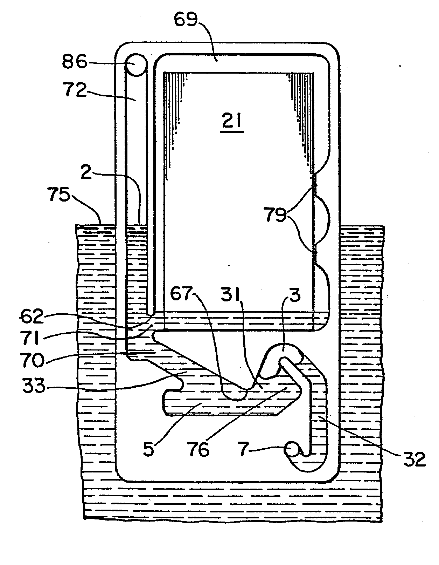

### DETAILED DESCRIPTION OF THE DRAWING

The surfactant cake 21 is isolated. The dispenser has vent hole 86, vent tube 72, cake compartment 69, outlet 71, baffle 67, passageway 70, bubble lock 3, air trap 76, reservoir 5, and exit port 7.

The protrusion 79 is there to steady the cake. The baffle means 67 must be designed to limit the size of the air lock bubble. The bubble 3 must be small enough to allow the dose volume of liquid above the baffle 67 to be discharged.

Note that inlet/outlet exit port 7 leads to first inlet/outlet passage way 32, which is in fluid communication with the bubble lock 3, which is in fluid communication with second inlet/outlet passageway 31 on the internal reservoir 5 side. Incline 33 of passageway 70 is sufficiently steep to shear excess air and vent same during refill. Passageway 70 leads to cake compartment 69 and vent tube 72.

The present invention is understood more clearly in the light of this disclosure and commonly owned U.S. Pat. No. 4,305,162, to Cornelisse, Jr., et al., issued December 15, 1981, for "Passive Dosing Dispenser Enveloping Captive Air Bubble to Provide Product Isolation," incorporated herein by reference.

The received water is routed to compartment 69 within the dispenser which contains the active. The dispenser has a lowermost edge 62 means for immersing only a lowermost portion of the cake to a predetermined depth in the received water to facilitate dissolving a portion of the cake for dispensing at a later time.

The liquid in the compartment 69 is drawn out each time the dispenser is fired, responsive to the flushing of the toilet. The quantity of fluid subject to discharge during the flush cycle is preferably 5 mls and the volume of solution in the dispenser including reservoir 5 is preferably from 10 to 12 mls. Reservoir 5 is shallow so that the liquid will sweep out insolubles with each flush.

When the toilet is flushed, the external water level 75 in the toilet tank drops, causing water level 2 to drop. The air bubble 3 moves out exit port 7. The volume of liquid above baffle 67 is released from the dispenser. As soon as the liquid level falls from level 2 to the baffle 67, the discharge action is terminated. None of the liquid volume below baffle 67 will be discharged.

### The Examples

Some preferred embodiments of the invention are illustrated by the following examples.

In the examples and the tables below, unless otherwise stated, all NaAS references mean essentially sodium lauryl sulfate.

0122664

- 12 -

EXAMPLE I

Steps for making a dry mix formulation having the salt to surfactant stoichiometric ratio of 0.5:1:

1. Put into an amalgamator:

| Ingredients | Parts |
| --- | --- |
| NaAS | 82.69 |
| MgSO$_4$ | 17.31 |
| | 100.00 |

2. Mix until uniform.

3. No optional ingredients are added to this one.

4. Charge about 65 grams of the dry mix into a die box having a rectangular face dimension of 4.3 cms x 8.6 cms.

5. Apply (using a Carver Press) 5,000 psi to the die box to form a cake.

6. This cake has dimensions of about 8.6 x 4.3 x 1.7 cms.

The cake is placed in an automatic dispensing device of the type described in the drawing. The device is suspended in the flush tank of a toilet, using a hanger device of the type described in U.S. Pat. No. 4,247,070, Dirksing, issued January 27, 1981, incorporated herein by reference. The cake has improved chemical stability over comparable cakes made with "a solid alkali earth metal surfactant." The cake of Example I also has improved longevity over cakes made with the alkali metal surfactant and only an alkali metal salt in place of the alkali earth metal salt. The cake performs satisfactorily in the device in that proper quantities of the composition are repeatedly delivered to the flush water in response to the flushing of the toilet.

EXAMPLE II

A preferred cake is made using the 76.3 parts of dry mix of Step 2 of Example I following the Suggested Steps outlined above in the Specification. Final formulation:

| Ingredients | Parts |
| --- | --- |
| NaAS | 63.09 |
| $MgSO_4$ | 13.21 |
| Perfume[1] | 18.00 |
| Fatty Alcohol[2] | 3.00 |
| Dye[3] | 2.70 |
| | 100.00 |

1. A pine fragrance perfume.
2. A $C_{12}$-$C_{16}$ fatty alcohol.
3. Acid Blue No. 9 dye.

After cutting the composition of this example into an 8.6 x 4.9 x 1.3 cm cake, it was placed in a dosing dispenser as in Example I and tested. The cake of this example is very stable upon storage and upon usage compared to cakes made with magnesium alkyl sulfate. The pH of this cake is about 7.5 in a 1% solution. The cake lasted for about 10 weeks with an average of about 10 flushes of the toilet per day, which is about twice the longevity of comparable prior art cakes made essentially with alkali metal salts and sodium alkyl sulfate.

- 1 -                    0122664

<u>CLAIMS</u>

1. A surfactant cake composition comprising: a dry mix of (A) a water-soluble alkali earth metal salt and (B) an anionic surfactant which is a water-soluble alkali metal salt of an organic sulfuric acid reaction product having in its molecular structure an alkyl chain containing from about 8 to about 22 carbon atoms and a radical selected from the group consisting of sulfonic acid and sulfuric acid ester radicals; said alkali earth metal salt and anionic surfactant being present in a salt:surfactant stoichiometric ratio of from about 0.1:1 to about 2:1.

2. The invention of Claim 1 wherein said ratio is from about 0.3:1 to about 1:1.

3. The invention of Claim 1 wherein said ratio is from about 0.5:1 to about 1:1.

4. The invention of Claim 1 wherein said alkali earth metal salt is magnesium sulfate and said surfactant is sodium alkyl sulfate wherein the alkyl chain contains from about 10 to about 16 carbon atoms.

5. The invention of Claim 1 wherein said alkali earth metal salt is selected from the group consisting of sulfates, chlorides and nitrates of magnesium, calcium, and barium.

6. The invention of Claim 1 wherein said cake comprises 20% up to 100% of said dry mix, 0% to 30% perfume, and 0% to 12% dye; said cake weighing from 10 gms to 120 gms and having a density of 0.8 to 1.5 gms/cc.

7. The invention of Claim 1 wherein said cake comprises 50% to 90% of said dry mix, 10% to 20% perfume, and 1.5% to 10% dye; said cake weighing from 20 gms to 100 gms and having a density of 1 to 1.3 gms/cc.

8. The invention of Claim 1 wherein said anionic surfactant is an alkali metal alkyl sulfate.

9. The invention of Claim 8 wherein said alkyl sulfate contains from about 10 to about 16 carbon atoms in its alkyl chain.

10. The invention of Claim 1 wherein a 1% solution of said cake has a pH of 7 to 9.5.

11. The invention of Claim 1 wherein said cake has a moisture content of 0.1% to 10%.

12. The invention of Claim 7 wherein the said cake has a moisture content of from about 1% to about 5%.

13. The invention of Claim 1 wherein said cake is made by admixing 50% to 90% of said dry mix wherein the stoichiometric ratio of alkali earth metal salt to said surfactant is from about 0.1:1 to 1:1;, from 0% to 15% free fatty alcohol, from 0% to 30% perfume, and from 0% to 12% dye; and compressing said admix to form a cake weighing from 10 gms to 120 gms, said cake having a density of 0.8 to 1.5, said cake having a pH of 6 to 10, said cake containing at least 1.5% of said perfume or said dye.

14. The invention of Claim 13 wherein said free fatty alcohol has carbon chain length of 8 to 18.

15. The invention of Claim 13 wherein said cake contains from 1% to 10% free fatty alcohol.

16. The invention of Claim 13 wherein said cake contains at least 10% of a combination of perfume, dye and a compatible inert water-soluble salt.

17. The invention of Claim 16 wherein said cake contains at least 50% of said dry mix and said stoichiometric ratio is about 0.5:1.

18. The invention of Claim 17 wherein said composition contains from 2% to 6% free fatty alcohol and from 50% to 90% of said dry mix.